# EUROPEAN PATENT APPLICATION

(11) **EP 1 630 731 A1**
(43) Date of publication of application: **01.03.2006**
(21) Application number: 03768224.2
(22) Date of filing: 25.12.2003
(51) Int. Cl.: G06Q 10/00

(54) **MEDICAL DEVICE RENTAL SYSTEM**

(30) Priority: 03.06.2003 JP 2003158461
(71) Applicant: Olympus Corporation, Tokyo 151-0072 (JP)
(72) Inventor: NOGUCHI, Toshiaki, Tachikawa-shi, Tokyo 190-0002 (JP); KUROSHIMA, Hisashi, Hachioji-shi, Tokyo 193-0832 (JP); SUZUKI, Eiri, Sagamihara-shi, Kanagawa 229-0038 (JP); HASEGAWA, Hitoshi, Yokohama-shi, Kanagawa 222-0002 (JP); GOCHO, Masanori, Hachioji-shi, Tokyo 192-0045 (JP)
(74) Representative: von Hellfeld, Axel
(86) International application number: PCT/JP2003/016691
(87) International publication number: WO 2004/109572

(57) **Abstract**

The medical equipment rental system comprises a plurality of hospitals, and a medical equipment rental center run by a rental operator who has concluded medical equipment rental agreements with these hospitals, the hospitals and the medical equipment rental center being interconnected by means of a communications line. The medical equipment rental center dispatches endoscope equipment required by a hospital, from a stock room, on the specified date and time, recovers the equipment from the examination room of the hospital after the examination, and cleans and sterilises the endoscope device that has been rented out, in a reprocessing room, before subsequently storing the equipment in a stock room. As a result of this, by renting out medical equipment requested by a rentee system, to the rentee system, by means of a rental service system on a fee-paying basis, it is possible to support medical examinations which are efficient and inexpensive.

## Description

### Technical Field

The present invention relates to a medical equipment rental system for renting medical equipment to medical organisations on a fee-paying basis.

### Background Art

In recent years, examinations and consultations using endoscopes have become widely used, regardless of the size of the hospital involved. By means of endoscopic examination, an affected area inside a patient's body cavity can be observed by a non-invasive method and a biopsy can readily be taken of a region of body tissue giving cause for concern, and hence this type of examination plays a very important role at both the stages of initial diagnosis and definitive diagnosis.

However, for hospitals where endoscopic examinations are carried out, there currently exist many problems such as those described below.
(1-1) Endoscopic systems are very expensive and the purchase of same represents a huge burden in budgetary terms for small hospitals, in addition to which, the reprocessing devices (for cleaning, disinfecting and sterilizing the equipment) is also very expensive and the purchase of same also represents a huge budgetary burden for small hospitals.
(1-2) Skilled specialist personnel are required in order to reprocess endoscopes correctly and ensure operator safety.
(1-3) Reprocessing of endoscopes requires a large amount of labour hour.
(1-4) It is difficult to hold a sufficient space for installing the reprocessing devices.
(1-5) If endoscopes are to be used for investigating different cases, then since the reprocessing time is long, it is necessary to prepare a large number of endoscopes.
(1-6) Even for a large hospital that deals with many different cases, preparing a large number of endoscopes presents a large burden, both in terms of the budget and the storage space required, and therefore, the number of cases that can be handled in one day is limited.
(1-7) The endoscopes require maintenance and inspection, such as leak tests, and the like.
(1-8) There may be types of endoscope that a hospital would like to have, but which are only used rarely, and which therefore cannot be purchased due to budgetary constraints.
(1-9) Budgetary constraints make it impossible to replace existing equipment with endoscope products newly released.

On the other hand, for patients receiving an endoscopic examination, the following types of problems exist.
(2-1) The patients feel insecure for the fact that the reprocessing history of the endoscopes used is unknown.
(2-2) The patients are sometimes obliged to travel to a large, distantly located hospital which is equipped with a line of endoscopic systems in order to undergo an endoscopic examination.

In order to resolve these problems, conventionally, reprocessing operations, such as cleaning, disinfecting and sterilization of equipment in the hospital, are subcontracted to an external operator for reprocessing. Such subcontracted reprocessing operations are mostly carried out by the subcontractor inside the hospital, whereas a part of the operations is carried out by taking the equipment out of the hospital.

However, it is not possible to resolve all of the problems described above by subcontracting the reprocessing operations. In other words, it is still necessary to purchase the endoscopic equipment, and if reprocessing is carried out in the hospital, then reprocessing equipment must also be purchased and installed. Furthermore, there also remains the problem that new products cannot be used readily, and the like.

It is an object of the present invention to provide a medical equipment rental system which is able to support efficient and inexpensive medical examinations, by enabling medical equipment requested by a rented system to have the rented system rented out from a rental service system, on a fee-paying basis.

### Disclosure of Invention

The medical equipment rental system according to the present invention comprises: a rentee system having: an examination category input section for inputting a category of a medical examination on the basis of diagnosis results; an examination date and time specifying section for specifying an examination date and time for the medical examination; and a rental order generating section for generating an order for rental of medical equipment required in the medical examination at the date and time specified by the examination date and time specifying section; and a rental service system having: a medical equipment securing section for securing the medical equipment required in accordance with an order generated by the rental order generating section; a medical equipment dispatching section for dispatching the medical equipment secured by the equipment securing section, to the requesting hospital, at the date and time specified by the examination date and time specifying section; and a reprocessing section for recovering the medical equipment after rental has terminated and reprocessing the medical equipment.

Other features and advantages of the present invention will become sufficiently apparent from the following description of the invention.

### Brief Description of the Drawings

Fig. 1 to Fig. 7 relate to a first embodiment of the present invention, wherein
Fig. 1 is a diagram showing the composition of a medical equipment rental system;
Fig. 2 is a diagram showing the composition of the hospital and the medical equipment rental center illustrated in Fig. 1;
Fig. 3 is a first diagram for describing the action of the medical equipment rental system illustrated in Fig. 1;
Fig. 4 is a second diagram for describing the action of the medical equipment rental system illustrated in Fig. 1;
Fig. 5 is a third diagram for describing the action of the medical equipment rental system illustrated in Fig. 1;
Fig. 6 is a fourth diagram for describing the action of the medical equipment rental system illustrated in Fig. 1; and
Fig. 7 is a fifth diagram for describing the action of the medical equipment rental system illustrated in Fig. 1.

### Best Mode for Carrying Out the Invention

In order to provide a more detailed understanding of the present invention, it is described below with reference to the accompanying drawings.

### First Embodiment

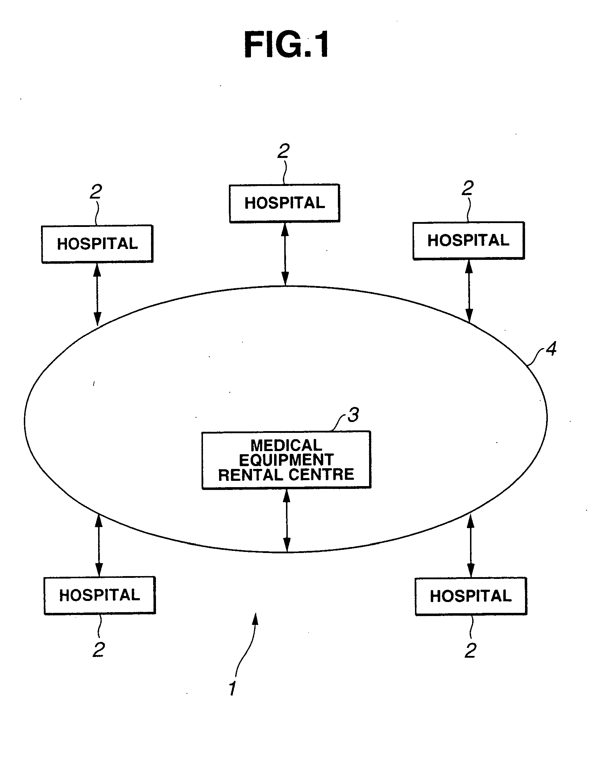

As shown in Fig. 1, the medical equipment rental system 1 according to the present embodiment comprises a plurality of hospitals 2, and a medical equipment rental center 3 run by a rental operator who has concluded medical equipment rental agreements with these hospitals 2, the hospitals 2 and the medical equipment rental center 3 being connected by means of a communications line 4. In the present embodiment, the rental of medical equipment is described below by taking an endoscope device as an example.

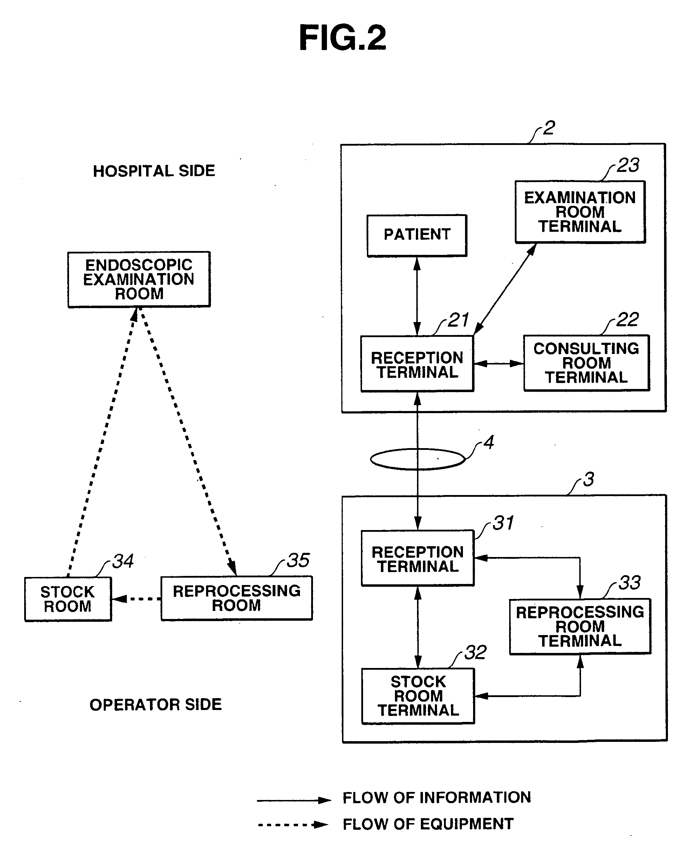

As shown in Fig. 2, a hospital 2 (or rather, the rentee system provided therein,) is provided with: a reception area terminal 21 for inputting information relating to a patient that has arrived at the hospital and the condition of that patient, and indicating the relevant consultation department to the patient; a consulting room terminal 22, disposed inside a consulting room of the relevant department, for receiving information about the patient and information about the patient's condition from the reception area terminal 21 and creating electronic patient records after the consultation; and an examination room terminal 23, disposed in an examination room where required endoscopic examination is carried out on a patient who has completed a consultation, for transmitting an examination schedule and examination results to a reception area terminal 21.

On the other hand, the medical equipment rental center 3 (or rather, the rental service system provided therein,) comprises: a reception terminal 31 for receiving requests from a reception area terminal 21 in a hospital 2, via the communications line 4, and managing the dispatch and return delivery of rented endoscope devices; a stock room terminal 32 for managing the stock of endoscope devices available for rental; and a reprocessing room terminal 33 for managing the reprocessing of endoscope devices that have been recovered after rental.

According to this composition, when a hospital 2 (or rather, the rentee system provided therein) makes a request to rent an endoscope device, the medical equipment rental center 3 (or rather, the rental service system provided therein) dispatches an endoscope device of the kind required by the hospital from a stock room 34, at the specified date and time, recovers the endoscope device from the examination room of the hospital 2 after examination, and cleans and sterilises the endoscope device that has been rented out, in a reprocessing room 35, before subsequently storing it in the stock room 34.

The detailed action of the present embodiment is described below.

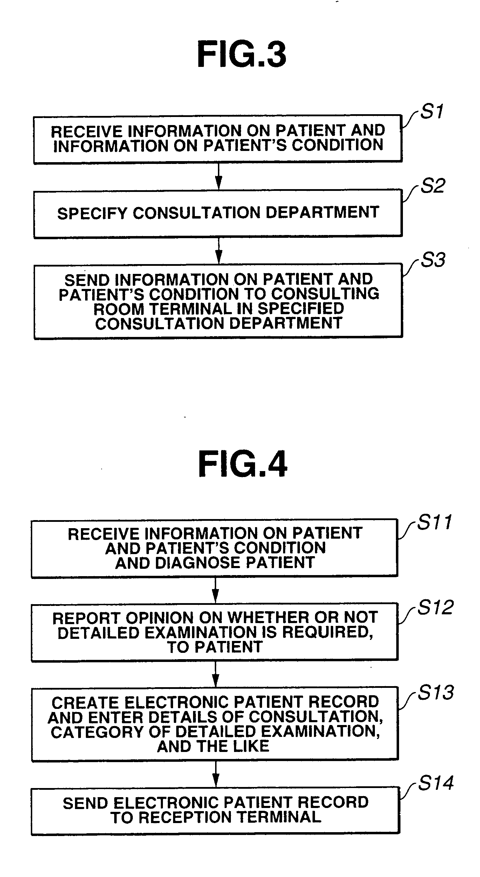

As illustrated in Fig. 3, when a patient arrives at a hospital 2, at step S1, information about the patient and information about the patient's condition are input to the reception area terminal 2, and at step S2, a consultation department that corresponds to the patient's particular condition is designated to the patient. At step S3, the information about the patient and the information about the patient's condition are sent by the reception area terminal 2 to the consulting room terminal 22 of the designated consultation department. With this, the reception procedure for the patient is completed.

In the consulting room of the designated consultation department, as shown in Fig. 4, at step S11, information about the patient and information about the patient's condition are received from the reception area terminal 2 by the consulting room terminal 22, and a consultation and diagnosis of the patient's condition are carried out. Thereupon, at step S12, the patient is informed whether or not he or she requires an endoscopic examination (detailed examination), and is also told the doctor's opinions (the results of the diagnosis), whereupon at step S13, the details of the diagnosis, and the endoscopic examination (detailed examination) category, and the like, are entered into an electronic patient record in the consulting room terminal 22, thereby creating an electronic patient record, which is sent to the reception area terminal 2 at step S14, whereby the consultation procedure for the patient is completed.

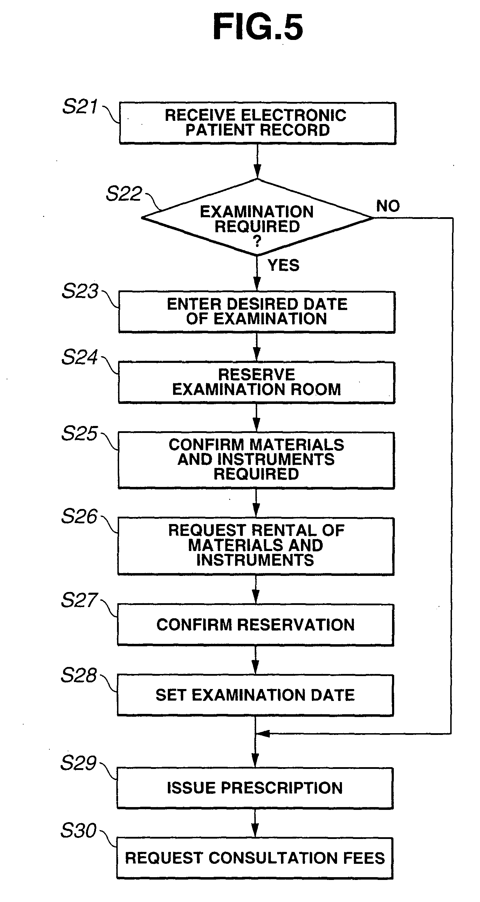

When the consultation has finished, the reception area terminal 2 receives the electronic patient record from the consulting room terminal 22 at step S21, as illustrated in Fig. 5, and at step S22, it is decided whether or not an endoscopic examination (detailed examination) is required. If an endoscopic examination (detailed examination) is required, then at step S23, the desired date of the examination for that patient is input, and at step S24, an examination room is reserved on the examination room terminal 23, in addition to which, at step S25, the materials and instruments required in the examination are confirmed. By means of this processing, it is possible to confirm the days on which examination is possible, and the materials and instruments required for the examination.

At step S26, a request for the materials and instruments required in the examination is made to the reception terminal 31 of the medical equipment rental center 3, by means of the communications line 4, and when a rental reservation has been confirmed by the reception terminal 31 of the medical equipment rental center 3 at step S27, then at step S28 the examination date is established. Thereupon, at step S29, a prescription, or the like, is issued to the patient, and at step S30, the patient is charged the consultation fee. If there is no requirement for an endoscopic examination (detailed examination), then the sequence proceeds from step S22 to step S29. Thereby, the discharge procedure for the patient is completed.

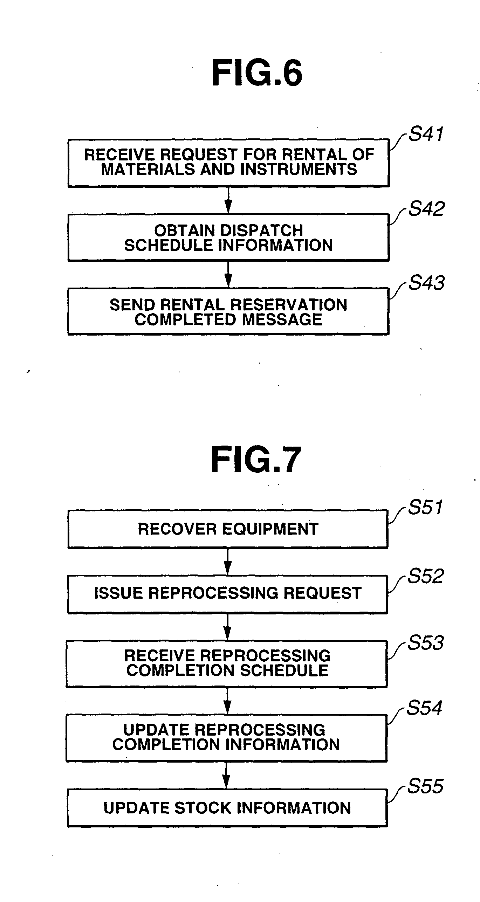

In the medical equipment rental center 3, on the other hand, as illustrated in Fig. 6, if a request for rental of materials and instruments is received from the reception area terminal 21 of the hospital 2 by means of the communications line 4 at step S41, then at step S42 dispatch schedule information is obtained from the stock room terminal 32, and at step S43, a rental reservation completed message is sent to the reception area terminal 21 of the hospital 2.

Here, it is also possible to make reservations in respect of a request for rental of materials and instruments from the reception area terminal 21 of the hospital 2, by means of the hospital making detailed specifications regarding the materials and instruments required, but it is also possible to receive reservations on the basis of an examination category correspondence table as illustrated in Table 1, by receiving the examination category from the hospital.

**(Table 1)**

| Category of Examination | Type of endoscope used | Peripheral equipment | Accessories |
|---|---|---|---|
| Endoscopic examination of upper digestive tract | GIF type scope | GIF peripheral set | GIF forceps set |
| Endoscopic examination of lower digestive tract | CF type scope | CF peripheral set | CF forceps set |

| | | | |
|---|---|---|---|
| Endoscopic examination of bronchus | BF type scope | BF peripheral set | BF forceps set |
| Endoscopic examination of biliary tract | JF type scope | JF peripheral set | JF forceps set |
| Endoscopic examination of lower digestive tract (Long) | CF type scope | CF peripheral set | CF forceps set |
| ... | ... | ... | ... |

Furthermore, in the medical equipment rental center 3, as illustrated in Fig. 7, when a rental has finished and the reception terminal 31 has confirmed the return of the materials and instruments that was rented out, at step S51, it then sends a request for reprocessing of the returned materials and instruments, to the reprocessing room terminal 23, at step S52. Thereupon, at step S53, it receives a reprocessing completion schedule from the reprocessing room terminal 23, and the materials and instruments are then transported to the reprocessing room 35. When reprocessing of the materials and instruments has been completed, at step S54, the reprocessing completed information is updated, and the materials and instruments that have completed reprocessing are conveyed to the stock room 34, where, at step S55, stock information is obtained from the stock room terminal 32 and the stock information is updated accordingly.

In this way, in the present embodiment, it is possible to provide medical equipment required by a hospital, on the date and time that it is required, and since the hospital does not need to carry out reprocessing of the equipment, then it is possible to support medical examinations that are efficient and inexpensive to run.

In this invention, it is obviously possible to compose various different modes of implementation covering a broad range of applications, on the basis of the present invention, without departing from the spirit or scope of the invention. This invention is limited by the accompanying claims, but apart from this, it is not limited by the specific embodiments described herein.

### Industrial Applicability

In this way, the medical equipment rental system according to the present invention is useful as a system for renting out medical equipment to medical organizations on a fee-paying basis.

## Claims

1. A medical equipment rental system, **characterized by** comprising:
a rentee system having: an examination category input section for inputting a category of a medical examination on the basis of diagnosis results; an examination date and time specifying section for specifying an examination date and time for the medical examination; and a rental order generating section for generating an order for rental of medical equipment required in the medical examination at the date and time specified by the examination date and time specifying section; and
a rental service system having: a medical equipment securing section for securing the medical equipment required in accordance with an order generated by the rental order generating section; a medical equipment dispatching section for dispatching the medical equipment secured by the equipment securing section, to a requesting hospital, at the date and time specified by the examination date and time specifying section; and a reprocessing section for recovering the medical equipment after rental has terminated and reprocessing the medical equipment.

2. The medical equipment rental system according to claim 1, **characterized in that** the rental order generating section of the rentee system comprises:
a reservation information generating section for generating reservation information including the date and time information specified by the examination date and time specifying section and information about the medical equipment required in the medical examination; and
a reservation information transmission section for transmitting the reservation information generated by the reservation information generating section, to the rental service system.

3. The medical equipment rental system according to claim 2, **characterized in that** the medical equipment securing section of the rental service system comprises:
a reservation information receiving section for receiving the reservation information from the reservation information transmission section;
a reservation judging section for judging whether or not the required medical equipment can be secured at the date and time specified by the examination date and time specifying section; and
a reservation acceptance information transmission section for transmitting information indicating that acceptance of a reservation has been completed, to the rentee system, in cases where the reservation judging section has judged that the required medical equipment can be secured at the date and time specified by the examination date and time specifying section.

4. The medical equipment rental system according to claim 1, **characterized in that** the medical equipment securing section of the rental service system comprises:
an examination category correspondence storing section for storing an examination category correspondence table which associates categories of medical examination with types of medical equipment;
the medical equipment required in accordance with the order generated by the rental order generating section being secured by referring to this examination category correspondence table.

5. The medical equipment rental system according to claim 2, **characterized in that** the medical equipment securing section of the rental service system comprises:
an examination category correspondence storing section for storing an examination category correspondence table which associates categories of medical examination with types of medical equipment;
the medical equipment required in accordance with the order generated by the rental order generating section being secured by referring to this examination category correspondence table.

6. The medical equipment rental system according to claim 3, **characterized in that** the medical equipment securing section of the rental service system comprises:
an examination category correspondence storing section for storing an examination category correspondence table which associates categories of medical examination with types of medical equipment;
the medical equipment required in accordance with the order generated by the rental order generating section being secured by referring to this examination category correspondence table.

7. A medical equipment rental method, **characterized by** comprising:
a rentee method having: an examination category inputting step of inputting a category of a medical examination on the basis of diagnosis results; an examination date and time specifying step of specifying an examination date and time for the medical examination; and a rental order generating step of generating an order for rental of medical equipment required in the medical examination at the date and time specified in the examination date and time specifying step; and
a rental service method having: a medical equipment securing step of securing the medical equipment required in accordance with an order generated in the rental order generating step; a medical equipment dispatching step of dispatching the medical equipment secured in the equipment securing step, to the requesting hospital, at the date and time specified in the examination date and time specifying step; and a reprocessing step of recovering the medical equipment after rental has terminated and reprocessing the medical equipment.

8. The medical equipment rental method according to claim 7, **characterized in that** the rental order generating step comprises:
a reservation information generating step of generating reservation information including date and time information specified in the examination date and time specifying step.

9. The medical equipment rental method according to claim 8, **characterized in that** the medical equipment securing step comprises:
a reservation information receiving step of receiving the reservation information generated in the reservation information generating step;
a reservation judging step of judging whether or not the required medical equipment can be secured at the date and time specified in the examination date and time specifying step; and
a reservation acceptance information generating step of generating information indicating that acceptance of a reservation has been completed, in cases where it has been judged in the reservation judging step that the required medical equipment can be secured at the date and time specified in the examination date and time specifying step.

10. The medical equipment rental method according to claim 7, **characterized in that** the medical equipment securing step secures the medical equipment required in accordance with the order generated in the rental order generating step, by referring to an examination category correspondence table which associates categories of medical examination with types of medical equipment.

11. The medical equipment rental method according to claim 8, **characterized in that** the medical equipment securing step secures the medical equipment required in accordance with the order generated in the rental order generating step, by referring to an examination category correspondence table which associates categories of medical examination with types of medical equipment.

12. The medical equipment rental method according to claim 9, **characterized in that** the medical equipment securing step secures the medical equipment required in accordance with the order generated in the rental order generating step, by referring to an examination category correspondence table which associates categories of medical examination with types of medical equipment.

13. An endoscope equipment rental system, **characterized by** comprising:
a rentee system having: an endoscopic examination category input section for inputting a category of an endoscopic examination on the basis of diagnosis results; an examination date and time specifying section for specifying an examination date and time for the endoscopic examination; and a rental order generating section for generating an order for rental of endoscope equipment required in the endoscopic examination at the date and time specified by the examination date and time specifying section; and
a rental service system having: an endoscope equipment securing section for securing the endoscope equipment required in accordance with an order generated by the rental order generating section; an endoscope equipment dispatching section for dispatching the endoscope equipment secured by the equipment securing section, to the requesting hospital, at the date and time specified by the examination date and time specifying section; and a reprocessing section for recovering the endoscope equipment after rental has terminated and reprocessing the endoscope equipment.

14. The endoscope equipment rental system according to claim 13, **characterized in that** the rental order generating section of the rentee system comprises:
a reservation information generating section for generating reservation information including the date and time information specified by the examination date and time specifying section and information about the endoscope equipment required in the endoscopic examination; and
a reservation information transmission section for transmitting the reservation information generated by the reservation information generating section, to the rental service system.

15. The endoscope equipment rental system according to claim 14, **characterized in that** the endoscope equipment securing section of the rental service system comprises:
a reservation information receiving section for receiving the reservation information from the reservation information transmission section;
a reservation judging section for judging whether or not the required endoscope equipment can be secured at the date and time specified by the examination date and time specifying section; and
a reservation acceptance information transmission section for transmitting information indicating that acceptance of a reservation has been completed, to the rentee system, in cases where the reservation judging section has judged that the required endoscope equipment can be secured at the date and time specified by the examination date and time specifying section.

16. The endoscope equipment rental system according to claim 13, **characterized in that** the endoscope equipment securing section of the rental service system comprises:
an examination category correspondence storing section for storing an examination category correspondence table which associates categories of endoscopic examination with types of endoscope equipment;
the endoscope equipment required in accordance with the order generated by the rental order generating section being secured by referring to this examination category correspondence table.

17. The endoscope equipment rental system according to claim 14, **characterized in that** the endoscope equipment securing section of the rental service system comprises:
an examination category correspondence storing section for storing an examination category correspondence table which associates categories of endoscopic examination with types of endoscope equipment;
the endoscope equipment required in accordance with the order generated by the rental order generating section being secured by referring to this examination category correspondence table.

18. The endoscope equipment rental system according to claim 15, **characterized in that** the endoscope equipment securing section of the rental service system comprises:
an examination category correspondence storing section for storing an examination category correspondence table which associates categories of endoscopic examination with types of endoscope equipment;
the endoscope equipment required in accordance with the order generated by the rental order generating section being secured by referring to this examination category correspondence table.

19. A medical equipment rental system, **characterized by** comprising:
a rentee system having: examination category input means for inputting a category of a medical examination on the basis of diagnosis results; examination date and time specifying means for specifying an examination date and time for the medical examination; and rental order generating means for generating an order for rental of medical equipment required in the medical examination at the date and time specified by the examination date and time specifying means; and
a rental service system having: medical equipment securing means for securing the medical equipment required in accordance with an order generated by the rental order generating means; medical equipment dispatching means for dispatching the medical equipment secured by the equipment securing means, to the requesting hospital, at the date and time specified by the examination date and time specifying means; and reprocessing means for recovering the medical equipment after rental has terminated and reprocessing the medical equipment.

20. An endoscope equipment rental system, **characterized by** comprising:
a rentee system having: endoscopic examination category input means for inputting a category of an endoscopic examination on the basis of diagnosis results; examination date and time specifying means for specifying an examination date and time for the endoscopic examination; and rental order generating means for generating an order for rental of endoscope equipment required in the endoscopic examination at the date and time specified by the examination date and time specifying means; and
a rental service system having: endoscope equipment securing means for securing the endoscope equipment required in accordance with an order generated by the rental order generating means; endoscope equipment dispatching means for dispatching the endoscope equipment secured by the equipment securing means, to the requesting hospital, at the date and time specified by the examination date and time specifying means; and reprocessing means for recovering the endoscope equipment after rental has terminated and reprocessing the endoscope equipment.
